Europäisches Patentamt

European Patent Office

Office européen des brevets

(11) Publication number: **0 287 924 B1**

# EUROPEAN PATENT SPECIFICATION

(45) Date of publication of patent specification: **09.09.92**    (51) Int. Cl.⁵: **F04D 33/00, A61M 1/10**

(21) Application number: **88105751.7**

(22) Date of filing: **12.04.88**

(54) **A pump by vessel revolution.**

(30) Priority: **22.04.87 JP 97436/87**

(43) Date of publication of application:
**26.10.88 Bulletin 88/43**

(45) Publication of the grant of the patent:
**09.09.92 Bulletin 92/37**

(84) Designated Contracting States:
**DE FR GB**

(56) References cited:
**DE-C- 486 575**
**GB-A- 771 840**

(73) Proprietor: **Fukui, Yasuhiro**
**52-10-1003, 5-chome Higashiikebukro**
**Toshima-ku**
**Tokyo(JP)**

Proprietor: **Miyashita, Osamu**
**2-5, 2-chome, Takakura**
**Iruma-shi Saitama-prefecture(JP)**

(72) Inventor: **Fukui, Yasuhiro**
**52-10-1003, 5-chome Higashiikebukro**
**Toshima-ku**
**Tokyo(JP)**
Inventor: **Miyashita, Osamu**
**2-5, 2-chome, Takakura**
**Iruma-shi Saitama-prefecture(JP)**

(74) Representative: **Schickedanz, Willi, Dipl.-Ing.**
**Langener Strasse 68**
**W-6050 Offenbach/Main(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Technical field to which the invention relates

The present invention relates to a pump by vessel revolution, comprising a vessel having an inlet for liquid. More particularly, the invention relates to a pump by vessel revolution suitable for blood circulation device such as an artificial heart or an assisted circulation or the like.

### Description of the prior art

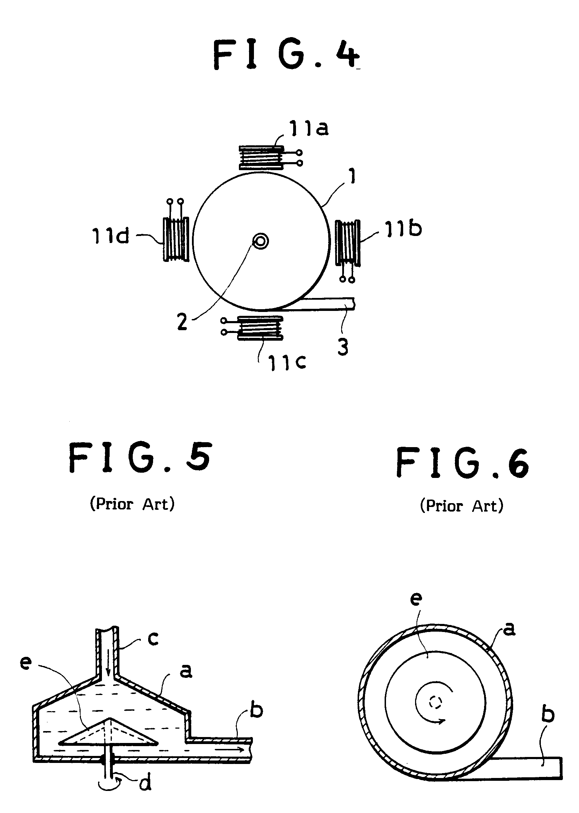

In the past, pumps of this kind have been known which are designed, for example, as shown in Figs. 5 and 6. More specifically, such a pump comprises a vessel (a), an outlet opening (b) provided in a tangential direction of an outer periphery at the lower part of the vessel (a), and an inlet (c) in the central portion at the upper part of the vessel (a). A sealed rotary shaft (d) is provided in the central portion at the lower part of the vessel (a), the rotary shaft (d) being extended within the vessel (a), an umbrella-like rotor (e) is secured to the end of the shaft, the rotor (e) being rotated by a drive device not shown from the bottom.

In the pump constructed as described above, the liquid flowing into the vessel (a) from the upper inlet (c) is rotated by the rotation of the rotor (e) and formed into a centrifugal flow which flows out of the outlet opening (b).

Still, a pump by vessel revolution is known which comprises a vessel having an inlet and an outlet for a liquid, whereby said vessel is designed in such a manner that the vessel can be revolved in a direction around an axis (DE-C-486 575).

Also, an improvement in rotary pumps of the inclined disc type is known which comprises an inlet duct for admitting fluid to be pumped to the center region of a casing and an outlet duct at the periphery of the casing and electromagnets to induce a swashing motion in the disc whereby fluid is pumped from the inlet to the outlet duct (GB-A-771 840).

### Disadvantages of the prior art

Accordingly, in the case where the pump as disclosed in Figs. 5 and 6 is used for heart or the like, the rotor rotates within the vessel and therefore various germs, air, foreign matters or the like are possibly mixed, which might cause hemolysis (destruction of red blood) or thrombus (condensation of blood). In addition, since a bearing for rotating the rotor is provided on the vessel, the durability of the bearing portion, particularly a seal of the bearing has disadvantages.

The disadvantage of the pump as disclosed by DE-C-486 575 lies in the fact that the liquid within the vessel rotates as a ring comprising a center of air.

As far as the pump according to GB-A-771 840 is concerned, an annular disc of soft iron is required which is located in a brass casing which provides two conic surfaces. Whereas the casing of this pump does not move, the annular disc moves due to the forces of electromagnets. The additional disc implies a complicated construction of the pump.

### Technical problem

The object of the present invention is to eliminate the need of a rotating means within the vessel and further to eliminate a seal of bearing for a rotary shaft of said rotating means accordingly.

### Solution of the technical problem

This above problem is solved in that an inlet is formed in the central portion of said vessel and a bugle-like membrane formed of a flexible synthetic resin is secured at its upper and lower ends to said vessel and arranged within said vessel, and a gas is sealed between said vessel and said membrane.

### Advantageous effects of the invention

According to the revolution of the vessel itself, the fluid flowing into the vessel from the inlet gives rise to a circular motion to produce a centrifugal flow. Since in the outer peripheral tangential direction, the outlet opening is provided in the same direction as the revolution, the liquid flows out of the vessel according to the revolving speed of the vessel. Therefore, when the pump is used for the device for circulating blood such as an artificial heart or assisted circulation, the blood is completely shut off from outside to free from entry of various germs, air, foreign matters or the like. Still, by means of the invention the construction can be simplified so as to provide high reliability and durability.

### Brief description of the drawings

Fig. 1    is a graph showing the relationship between the eccentric distance from the center of revolution of the vessel and the discharge pressure;

Fig. 2    is a partly cut away view of a first embodiment;

Fig. 3    is a partly cut away view of a second embodiment;

Fig. 4    is a plan view of a third embodiment; and

Figs.5 and 6   illustrate a conventional centrifugal pump.

## Description of preferred embodiments

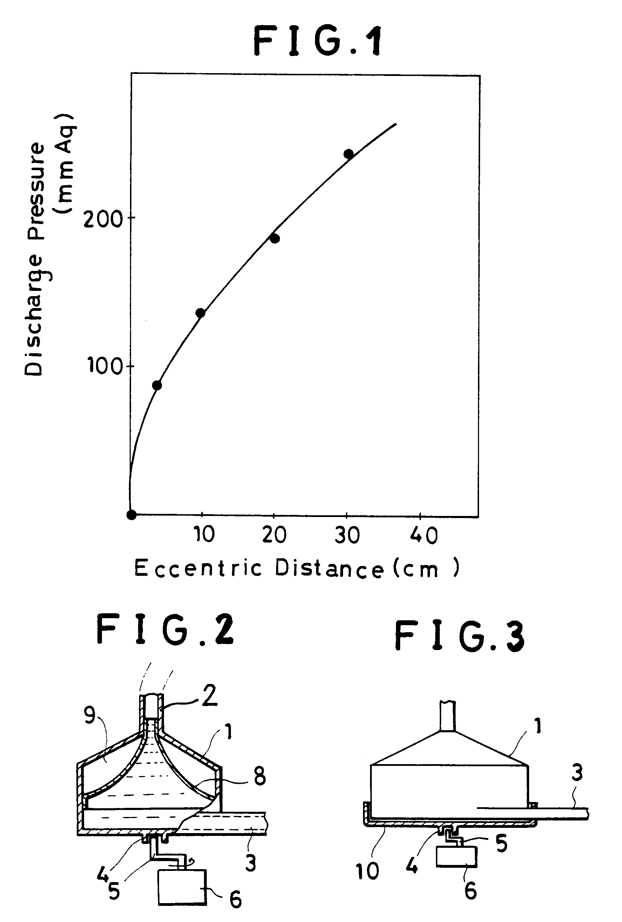

Fig. 1 shows the relationship between the eccentric distance form the center of revolution of a vessel and the discharge pressure as obtained by experiment. In this experiment the eccentric distance was variously changed to obtain the respective discharge pressure. In this experiment, the number of revolutions of the vessel was 960 r.p.m.

It has been proved from this graph that the discharge pressure increases as the eccentric distance increases.

Fig. 2 shows a first embodiment of the invention. A vessel indicated at 1, which is formed of synthetic resin or the like, has an inlet 2 in the vicinity of a center position at the upper part thereof and an outlet opening 3 in a tangential direction of an outer peripheral portion thereof, and is rotatably held by means such as a flexible pipe or a roller provided on the lower surface of the vessel. A projected recess 4 is provided in the lower surface of the vessel 1, and an electric motor 6 having a crank-like rotary shaft 5 loosely fitted into the recess 4 is provided at the lower part of the vessel 1, the rotational direction of the motor 6 being preset so as to be the same as the tangential direction of the outlet opening 3. The end of the inlet 2 and the end of the outlet opening 3 are connected by a flexible pipe or the like for free revolution of the vessel 1.

With the above-described arrangement, the liquid flows in from the inlet 2 of the vessel and the motor 6 is energized to rotate the shaft 5. Accordingly, the liquid within the vessel 1 gives rise to a circular motion to generate a centrifugal flow or eddy current and flows out of the outlet opening 3. The speed of the liquid to be flowed out is related to the speed of revolution of the vessel 1 and can be adjusted by the inside diameter of the outlet opening 3, the shape of a connecting portion with the vessel 1, and the like.

In this embodiment a bugle-like membrane 8 formed of a flexible synthetic resin material comprising a soft film is secured at upper and lower ends to the vessel 1, and air or other gas 9 is sealed in a space between the vessel 1 and the membrane 8. There can be obtained a freedom of a centrifugal flow region by the air 9 and the resiliency of the membrane 8 in a centrifugal flow of liquid with the revolution of the vessel, thus effectively producing a centrifugal flow to enhance the discharge performance.

Fig. 3 shows a second embodiment, in which the vessel 1 is supported by a tray-like support member 10 having at the lower surface thereof a recess 4 loosely fitted into the shaft 5. This can be conveniently applied to the case where the vessel 1 is formed of a material of inconvenience in processing accuracy, mechanical strength or the like such as glass, synthetic resin or the like.

While in any of the aforementioned embodiments, the end of the shaft 5 is loosely fitted into the recess 4 in the lower surface of the vessel 1 or the support body 10, it is to be noted that reversely to the former, a recess is provided in the end of the shaft so that a projection projected on the lower surface of the vessel 1 or the support body 10 is loosely fitted into the recess, or in place of the recess and shaft or the loose fit of the projection, both the elements can be connected by rotatable connecting means such as a bearing.

Fig. 4 shows a third embodiment, which shows another example of a driving device for revolving the vessel 1, in which the vessel 1 is formed of an electromagnetic material such as permalloy and provided on the lower surface.

Electromagnetic coils 11a, 11b, 11c and 11d are provided opposedly to the side of the vessel 1 at intervals, and when pulses from an oscillator are successively applied to the electromagnetic coils 11a, 11b, 11c,11d, 11a, 11b..., the vessel 1 is revolved. In this embodiment, the connecting mechanism such as the recess 4 and the shaft 5 in the previously described embodiment is not provided, and therefore there occurs no mechanical wear,noises and the like, thus providing an excellent durability.

## Claims

1. A pump by vessel revolution, comprising
    1.1 a vessel (1)
        1.1.1 having an inlet (2) formed in the central portion of said vessel (1)
        1.1.2 and an outlet (3) in a tangential direction of an outer peripheral portion of said vessel (1);
    1.2 means (4 through 6) for revolving said vessel (1) around an axis;
    **characterized in that**
    1.3 a bugle-like membrane (8) formed of a flexible synthetic resin is secured at its upper and lower ends to said vessel (1) and arranged within said vessel (1);
    1.4 a gas (9) is seeled between said vessel (1) and said membrane (8).

2. A pump as defined in claim 1, **characterized in that** an electric motor (6) is provided below said vessel (1), and that an end of a crank-like rotary shaft (5) of said motor (6) is rotatably connected to the central portion of the lower portion of said vessel (1).

3. A pump as defined in claim 1, **characterized in that** the occupied ratio of gas (9) between said vessel (1) and said membrane (8) is eight to twelve percent.

4. A pump as defined in claim 1, **characterized in that** said vessel (1) is formed of an electromagnetic material, and a plurality of electromagnetic coils (11a through 11d) are disposed sideway of said vessel (1), said electromagnetic coils (11a through 11d) being successively energized pulsewise.

5. A pump as defined in claim 1, **characterized in that** the vessel (1) is supported by a tray-like support member (10) having at its lower surface a recess (4) loosely fitted into a shaft (5).

6. A pump as defined in claim 1, **characterized in that** a recess is provided in the end of a shaft so that a projection projected on the lower surface of the vessel (1) or a support member (10) is loosely fitted into said recess.

## Patentansprüche

1. Pumpe mit sich drehendem Behälter, enthaltend
   1.1 einen Behälter (1),
      1.1.1 mit einem Einlaß (2), der in der Mitte des Behälters (1) angebracht ist,
      1.1.2 und einem Auslaß (3), der in tangentialer Richtung eines äußeren Umfangsbereichs des Behälters (1) vorgesehen ist;
   1.2 eine Vorrichtung (4 bis 6), die den Behälter (1) um eine Achse dreht;
   **dadurch gekennzeichnet, daß**
   1.3 eine hornförmige Membran (8) aus flexiblem Kunststoff an ihren oberen und unteren Enden an dem Behälter(1) befestigt und in dem Behälter (1) angebracht ist;
   1.4 ein Gas (9) zwischen dem Behälter (1) und der Membran (8) luftdicht eingeschlossen ist.

2. Pumpe nach Anspruch 1**, dadurch gekennzeichnet,** daß ein Elektromotor (6) unter dem Behälter (1) vorgesehen ist und daß ein Ende einer kurbelförmigen Drehwelle (5) des Motors (6) drehbar mit dem mittleren Bereich des unteren Bereichs des Behälters (1) verbunden ist.

3. Pumpe nach Anspruch 1, **dadurch gekennzeichnet,** daß das Volumenverhältnis des Gases (9) zwischen dem Behälter (1) und der Membran (8) acht bis zwölf Prozent beträgt.

4. Pumpe nach Anspruch 1, **dadurch gekennzeichnet,** daß der Behälter (1) aus elektromagnetischem Material hergestellt ist und mehrere elektromagnetische Spulen (11a bis 11d) seitlich vom Behälter (1) angeordnet sind, wobei diese elektromagnetischen Spulen (11a bis 11d) nacheinander pulsweise mit Energie beaufschlagt werden.

5. Pumpe nach Anspruch 1, **dadurch gekennzeichnet,** daß der Behälter (1) von einem trogförmigen Halteelement (10) getragen wird, das auf seiner Unterseite eine Aussparung (4) aufweist, in die eine Welle (5) lose eingreift.

6. Pumpe nach Anspruch 1, **dadurch gekennzeichnet,** daß an dem Ende einer Welle eine Aussparung vorgesehen ist, so daß ein auf der Unterseite des Behälters (1) oder eines Halteelements (10) befindlicher Vorsprung lose in die Aussparung eingreift.

## Revendications

1. Pompe à baril tournant, comprenant
   1.1 un baril (1)
      1.1.1 possédant une ouverture (2) formée dans la portion centrale dudit baril (1)
      1.1.2 et une sortie (3) ménagée dans une direction tangentielle d'une portion périphérique extérieure dudit baril (1) ;
   1.2 des moyens (4 à 6) destinés à faire tourner ledit baril (1) autour d'un axe ;
   caractérisée en ce que
   1.3 une membrane en forme de trompette (8) constituée d'une résine synthétique souple est fixée à ses extrémités supérieure et inférieure audit baril (1) et disposée à l'intérieur dudit baril (1);
   1.4 un gaz (9) est enfermé hermétiquement entre ledit baril (1) et ladite membrane (8).

2. Pompe selon la revendication 1, caractérisée en ce qu'un moteur électrique (6) est disposé au-dessous dudit baril (1), et en ce qu'une extrémité d'un arbre rotatif en forme de manivelle (5) dudit moteur (6) est connectée en rotation à la portion centrale de la partie inférieure dudit baril (1).

3. Pompe selon la revendication 1, caractérisée en ce que le rapport de gaz (9) engagé entre ledit baril (1) et ladite membrane (8) est de huit à douze pour cent.

4. Pompe selon la revendication 1, caractérisée en ce que ledit baril (1) est constitué en matériau électromagnétique, et en ce qu'une pluralité de bobines électromagnétiques (11a à 11d) sont disposées sur le côté dudit baril (1), lesdites bobines électromagnétiques (11a à 11d) étant alimentées successivement en énergie par impulsions.

5. Pompe selon la revendication 1, caractérisée en ce que le baril (1) est soutenu par un élément de support en forme de plateau (10) présentant, sur sa surface inférieure, une rainure (4) fixée de façon amovible dans un arbre (5).

6. Pompe selon la revendication 1, caractérisée en ce qu'une rainure est disposée à une extrémité d'un arbre, de telle manière qu'une saillie dépassant sur la surface inférieure du baril (1) ou qu'un élément de support (10) soit fixé de façon amovible dans ladite rainure.

# FIG.1

Discharge Pressure (mm Aq) vs Eccentric Distance (cm)

# FIG.2

# FIG.3

# FIG.4

# FIG.5

(Prior Art)

# FIG.6

(Prior Art)